Europäisches Patentamt

European Patent Office

Office européen des brevets

(11) Publication number: **0 363 185**
**A1**

## (12) EUROPEAN PATENT APPLICATION

(21) Application number: **89310169.1**

(22) Date of filing: **04.10.89**

(51) Int. Cl.⁵: **B01J 35/06 , B01J 23/74**

(30) Priority: **04.10.88 ZA 885687**

(43) Date of publication of application:
**11.04.90 Bulletin 90/15**

(84) Designated Contracting States:
**AT BE CH DE ES FR GB GR IT LI LU NL SE**

(71) Applicant: **CRO CAT CC**
**Suite 7 Denavo Building 15 York Street**
**Kensington B Randburg Transvaal(ZA)**

(72) Inventor: **Gulumian, Mairam**
**Plot 53 Pine Road Kyalami**
**Midrand Transvaal(ZA)**
Inventor: **Copperthwaite, Richard George**
**14 3rd Avenue Parktown North**
**Johannesburg Transvaal(ZA)**

(74) Representative: **Perry, Robert Edward et al**
**GILL JENNINGS & EVERY 53-64 Chancery**
**Lane**
**London WC2A 1HN(GB)**

(54) **Catalysts.**

(57) Solid catalysts which comprise an iron - containing mineral fibre in which the iron concentration, as Fe exceeds 10% by mass based on the dry mineral fibre, for use in catalysing fluid-phase reactions. The chemical composition of the mineral fibre may be modified by physically or chemically removing some of the constituents occurring naturally in the mineral fibre, for example by acid leaching. The chemical composition of the mineral fibre may also be modified by adding additional constituents to the mineral fibre, for example, by impregnating metal oxides into the mineral fibre. The catalysts may also be mixed with suitable carriers to produce catalyst compositions.

EP 0 363 185 A1

## CATALYSTS

### BACKGROUND TO THE INVENTION

This invention relates to solid catalysts for catalysing fluid-phase reactions.

More particularly, the invention relates to a catalyst comprising an iron-containing mineral fibre, to a catalyst composition, to a method for the manufacture of a catalyst and to processes in which an iron-containing catalyst is used.

Metals, in particular iron and cobalt, are used as or in catalysts in the synthesis of hydrocarbons, oxygenates and ammonia from respectively, synthesis gas (in the case of hydrocarbons and oxygenates) and a mixture of nitrogen and hydrogen (in the case of ammonia).

In the case of hydrocarbon synthesis, such as for example in the Fischer-Tropsch synthesis, an iron catalyst is used. Such catalyst can be in the form of finely divided particles for use in the entrained bed or fluidised bed processes such as the Synthol process used by Sasol. Another example of a Fischer-Tropsch catalyst is one that is used in the fixed bed process. Such a catalyst is made of precipitated iron and copper in a silica matrix.

### SUMMARY OF THE INVENTION

According to the invention, a catalyst comprises an iron containing mineral fibre, in which the iron concentration, as Fe, exceeds 10% by mass based on dry mineral fibre.

The catalyst may be selected from the minerals crocidolite and amosite. In nature these minerals occur as bundles of fine parallel fibres, but for use as catalysts, the fibres are preferably teaseled.

The mineral fibre may be suitably modified so as to enhance some of its desirable properties or so as to suppress some of its undesirable properties. Thus, some of the constituents occurring naturally in the mineral fibre may be either chemically or physically removed therefrom. For example, at least some of the iron originally present in the mineral fibre, can be removed therefrom by acid leaching. Alternatively, or in addition, a suitable concentration of any one or more of the following may be introduced into the mineral fibre by, for example, impregnation : a metal, a metal oxide, a salt or an oxide of an element of group 1 or group 2 of the periodic table.

The chemical composition of mineral fibres is variable and fibres having suitable chemical and physical properties are selected. Thus, for synthesising hydrocarbons from synthesis gas or for synthesising ammonia, it is advantageous to select fibres having a suitable iron concentration which is sufficiently high even after leaching, by means of acid leaching, has been carried out.

The length and the diameter of the mineral fibres which are selected is determined by the type of reactor to be used as well as the process conditions present. The catalyst of the present invention may be used in entrained bed reactors, fixed bed reactors, fixed/fluidised bed reactors or constant stir tank reactors and under the following exemplary process conditions:
Temperature from about 150°C to about 450°C;
Pressure from about one atmosphere to about 30 atmospheres (gauge); and an $H_2$/CO ratio of between 0.5 to 3 (by Vol.).

Impregnation of the fibres may be done by precipitating iron hydroxide in the mineral fibre followed by conversion of the iron hydroxide to iron or iron oxide.

In its natural state, the iron occurring in amosite is mainly in the ferrous form (Fe (II)) and the iron occurring in crocidolite is both in the ferrous form (Fe (II)) and ferric form (Fe (III)).

In order to influence the catalytic activity of the mineral fibre, the oxidation state of the iron may be altered. Thus, for example, a portion of the iron in the mineral fibre may be converted to iron (II) oxide, whilst another portion may be converted from iron (II) oxide to iron (III) oxide or vice versa. In this context, an average oxidation state for the iron in the fibre may be determined and such average may for instance be used as an indication of the oxidation state of the iron present in the fibre.

To increase the average oxidation state, the mineral fibre may be calcined in air at a temperature of from 400°C to 500°C. Alternatively, in order to reduce the average oxidation state, the fibre may be treated with hydrogen at a temperature of from 200°C to 700°C.

In order to produce a catalyst composition, the catalyst may be mixed with a suitable binder, promoter, carrier and/or diluent. The composition may be prepared by way of extrusion in the form of pellets. Alternatively, the composition may be formed into particles having shapes which are aimed at optimising both pressure drop and efficiency of contact between the catalyst and the materials taking part in the reaction.

Further according to the invention, there is provided a process for the manufacture of hydrocarbons from synthesis gas, including the step of converting, under suitable conditions of temperature and pressure, the synthesis gas comprising hydrogen and carbon monoxide, in a suitable ratio of one to the other, over a catalyst comprising an iron-containing mineral fibre in which the iron concentration, as Fe, exceeds 10% by mass based on dry mineral fibre.

The invention also extends to a process for the manufacture of ammonia, including the step of converting, under suitable conditions of temperature and pressure, a gas mixture comprising hydrogen and nitrogen in a suitable ratio of one to the other, over a catalyst comprising an iron-containing mineral fibre, in which the iron concentration, as Fe, exceeds 10% by mass based on dry mineral fibre.

EXAMPLES

An UICC (Union Internationale Contre Le Cancer) standard reference sample of crocidolite was used for catalyst testing (Example 5). This consisted of fibres of length distribution 0.2 to 0.5 microns. The specific surface area, determined by nitrogen adsorption, was $8.0 m^2$ per gram. Commercially available crocidolite fibres were used for Examples 1-4.

In Examples 1-4 1.0 g of the crocidolite was placed in a stainless steel microreactor connected on-line to a gas chromatograph with both TCD and FID detectors. The pressure in the microreactor was maintained at either 1 bar (0.1 MPa), 3 bar (0.3 MPa), 9 bar (0.9 MPa) or 5 bar (0.5 MPa). Mass balances were obtained over approximately 5 days of continuous reaction. Gas products up to an including $C_{11}$ hydrocarbons were quantitively determined by standard procedures. (Dietz W.A., J of Gas Chromatography 1967,68).

## EXAMPLE 1

Catalyst : Asbestos (Crocidolite)-1 g (Vol 3.848 ml)
Run time : 144 hours
Reduction: GHSV = 210 $h^{-1}$ 420°C for 16 hours 1 bar (0.1 MPa) $H_2$

## CARBON MONOXIDE (CO) HYDROGENATION CONDITIONS

Run temperature : 250°C
GHSV : 317.72 $h^{-1}$
Pressure : 1 bar (0.1 MPa)
CO Conversion : 16.54 v/v %
$H_2$/CO ratio : 2 (by Vol.)

## HYDROCARBON SELECTIVITY (MASS %)

$C_1$ (Methane) 9.6
$C_2$ (Ethene) 3.5

$C_2$ (Ethane) 2.7
$C_3$ (Propene) 5.4
$C_3$ (Propane) 1.2
$C_4$ (Saturates and Unsaturates) 6.7
$C_5$ (Saturates and Unsaturates) 7.7
$C_6$ (Saturates and Unsaturates) 2.1
$C_7$ (Saturates and Unsaturates) 3.8
$C_8$ (Saturates and Unsaturates) 5.7
$C_9$ (Saturates and Unsaturates) 16.0
$C_{10}$ (Saturates and Unsaturates) 27.9
$C_{11}$ (Saturates and Unsaturates) 7.6
$C_2H_4/C_2H_6$ = 1.3
$C_3H_6/C_3H_6$ = 4.9

## EXAMPLE 2

Catalyst : Asbestos (Crocidolite) - 1 g (Vol. 3.848 ml)
Run Time : 119 hours
Reduction: GHSV = 210 $h^1$ 420°C for 16 hours 1 bar (0.1 MPa) $H_2$

## CARBON MONOXIDE (CO) HYDROGENATION CONDITIONS

Run temperature : 250°C
GHSV : 273.34 $h^{-1}$
Pressure : 3 bar (0.3 MPa)
CO conversion : 25.25 v/v%
$H_2$/CO ratio : 2 (by Vol.)

## HYDROCARBON SELECTIVITY (MASS %)

$C_1$ (Methane) 9.8
$C_2$ (Ethene) 1.8
$C_2$ (Ethane) 3.5
$C_3$ (Propene) 5.6
$C_3$ (Propane) 2.2
$C_4$ (Saturates and Unsaturates) 7.8
$C_5$ (Saturates and Unsaturates) 8.1
$C_6$ (Saturates and Unsaturates) 8.0
$C7$ (Saturates and Unsaturates) 9.6
$C_8$ (Saturates and Unsaturates) 17.6
$C_9$ (Saturates and Unsaturates) 11.3
$C_{10}$ (Saturates and Unsaturates) 4.7
$C_{11}$ (Saturates and Unsaturates) 10.0
$C_2H_4/C_2H_6$ = 0.5
$C_3H_6/C_3H_6$ = 2.6
Mass of wax : 3.7 g
Mass of organic liquid : 2.6 g
Mass aqueous liquid : 6.5 g
Wax : 0.6321 g melting range : 77-89°C
$CO_2$ = 2.6 v/v%

## EXAMPLE 3

Catalyst : Asbestos (Crocidolite) - 1 g (Vol. 3.848 ml)

Run time : 167 hours
Reduction: GHSV = 210 h⁻¹ 420°C for 16 hours 1
bar (0.1 MPa) $H_2$

## CARBON MONOXIDE (CO) HYDROGENATION CONDITIONS

Run temperature : 250°C
GHSV : 252.47 h⁻¹
Pressure : 9 bar (0.9 MPa)
CO conversion : 16.03 v/v%
$H_2$/CO ratio : 2 (by Vol.)

## HYDROCARBON SELECTIVITY (MASS %)

$C_1$ (Methane) 7.4
$C_2$ (Ethene) 3.6
$C_2$ (Ethane) 3.8
$C_3$ (Propene) 7.4
$C_3$.(Propane) 1.8
$C_4$ (Saturates and Unsaturates) 9.1
$C_5$ (Saturates and Unsaturates) 8.3
$C_6$ (Saturates and Unsaturates) 7.7
$C_7$ (Saturates and Unsaturates) 8.5
$C_8$ (Saturates and Unsaturates) 12.4
$C_9$ (Saturates and Unsaturates) 14.7
$C_{10}$ (Saturates and Unsaturates) 8.8
$C_{11}$ (Saturates and Unsaturates) 6.4
$C_2H_4/C_2H_6$ = 1.0
$C_3H_6/C_3H_6$ = 4.0
Mass of organic liquid : 1.2 g
Mass aqueous liquid : 1.9 g
$CO_2$ = 0.5 v/v%

## EXAMPLE 4

Catalyst : Asbestos (Crocidolite) - 1 g (Vol. : 3.848 ml)
Run time : 456 hours
Reduction : GHSV = 210 h⁻¹ 420°C for 16 hours 1 bar (0.1 MPa) $H_2$
Calcination: 500°C for 24 hours in air
Promotion : 5% K⁺

## CARBON MONOXIDE (CO) HYDROGENATION CONDITIONS

Run temperature : initially 300°C increased to 350°C after 5 days
GHSV : 288.31 h⁻¹
Pressure : 5 bar (0.5 MPa)
CO conversion : 70.76 v/v%
$H_2$/CO ratio : 2 (by Vol.)

## HYDROCARBON SELECTIVITY (MASS %)

$C_1$ (Methane) 23.6
$C_2$ (Ethene) 4.0

$C_3$ (Propene) 9.6
$C_3$ (Propane) 7.0
$C_4$ (Saturates and Unsaturates) 9.3
$C_5$ (Saturates and Unsaturates) 8.2
$C_6$ (Saturates and Unsaturates) 5.6
$C_7$ (Saturates and Unsaturates) 3.8
$C_8$ (Saturates and Unsaturates) 4.1
$C_9$ (Saturates and Unsaturates) 2.3
$C_{10}$ (Saturates and Unsaturates) 5.6
$C_{11}$ (Saturates and Unsaturates) 3.5
$C_2H_4/C_2H_6$ = 0.298
$C_3H_6/C_3H_6$ = 1.384
Mass of liquid = 6.3329 g

## EXAMPLE 5

2.0 g of the crocidolite was placed in a pyrex plug-flow reactor and hydrogen gas at 1 bar (0.1 MPa), flow rate 40 ml/min was passed through the reactor at a temperature of 400°C for 42½ hours. The hydrogen flow was then substituted with a synthesis gas mixture at 1 bar (0.1 MPa), composition by mass CO, 49%, $H_2$, 50%, $N_2$ 1%, flow rate 17 ml/min and the hydrocarbons produced were determined using a gas chromatograph fitted with both TCD and FID. At a reactor temperature of 220°C the gaseous composition downstream of the reactor after 28 hours on-line was:

## HYDROCARBON SELECTIVITY (MASS %)

$C_1$ (Methane) 27.1
$C_2$ (Ethene) 30.5
$C_2$ (Ethane) 1.8
$C_3$ (Propene) 25.2
$C_3$ (Propane) 0.6
$C_4$ (Saturates) 3.2
$C_4$ (Unsaturates) 8.3
$C_5$ (Saturates and Unsaturates) 2.4
$C_6$ (Saturates and Unsaturates) 0.7
CO conversion under these conditions was 12.5% (m/m). In order to suppress methane formation alkali or other promoters may be added to the bed.

## Claims

1. A catalyst comprising an iron-containing mineral fibre, in which the iron content, as Fe, exceeds 10% by mass based on dry mineral fibre.

2. A catalyst as claimed in claim 1 in which all of the iron content forms part of the structure of the mineral fibre.

3. A catalyst as claimed in claim 1 in which a portion of the iron content forms part of the structure of the mineral fibre and a portion of the iron content is dispersed on the surface of the mineral fibre.

4. A catalyst as claimed in claim 1 in which the mineral fibre is amosite.

5. A catalyst as claimed in claim 1 in which the mineral fibre is crocidolite.

6. A catalyst as claimed in any one of claims 1 to 5, in which at least a portion of the chemical composition the mineral fibre has been altered.

7. A catalyst as claimed in claim 6 in which a portion of the iron originally present in the mineral fibre has been removed therefrom by acid leaching.

8. A catalyst as claimed in any one of claims 1 to 7, comprising, in addition to the mineral fibre, a suitable concentration of any one or more of the following:
a metal, a metallic oxide, a metallic salt, a non-metallic element, a salt of a non-metallic element, or an oxide of a non-metallic element.

9. A catalyst as claimed in claim 6, in which an amount of iron or iron oxide is introduced into the mineral fibre by impregnation.

10. A catalyst as claimed in claim 9 in which the impregnation is carried out by precipitation of iron hydroxide in the mineral fibre and by conversion of the iron hydroxide to iron or iron oxides.

11. A catalyst as claimed in any one of claims 1 to 10 in which the oxidation state of at least some of the iron occurring naturally in the mineral fibre or of the iron present therein after pretreatment, has been increased.

12. A catalyst as claimed in claim 11, in which the oxidation state of the iron has been increased by calcination in air.

13. A catalyst as claimed in any one of claims 1 to 10 in which the oxidation state of at least some of the iron occurring naturally in the mineral fibre or of the iron present therein after impregantion has been decreased.

14. A catalyst as claimed in claim 13, in which the oxidation state of the iron has been decreased by hydrogen treatment at a temperature of from 200°C to 700°C.

15. A catalyst composition, comprising a catalyst as claimed in any one of claims 1 to 14 and a suitable binder, carrier and or diluent.

16. A process for the manufacture of hydrocarbons or oxygenated derivatives thereof including the step of converting, under suitable conditions of temperature and pressure, a synthesis gas comprising hydrogen and carbon monoxide in a suitable ratio of one to the other, over a catalyst comprising an iron-containing mineral fibre in which the iron concentration, as Fe, exceeds 10% by mass based on dry mineral fibre.

17. A process for the manufacture of ammonia, including the step of converting, under suitable conditions of temperature and pressure, a gas mixture comprising hydrogen and nitrogen in a suitable ratio of one to the other, over a catalyst comprising an iron-containing mineral fibre in which the iron concentration, as Fe, exceeds 10% by mass based on a dry mineral fibre.

European Patent Office

# EUROPEAN SEARCH REPORT

Application Number

EP 89 31 0169

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 034 596 (UNITED KINGDOM ATOMIC ENERGY AUTHORITY) <br> * Claims 1,11,19 * <br> --- | 1,2 | B 01 J 35/06 <br> B 01 J 23/74 |
| X | US-A-3 870 530 (G.J. ROBERTS) <br> * Claims 1,3; column 3, lines 62-68 * | 1,2 | |
| Y | | 6,8,9, 13-15 | |
| | --- | | |
| Y | FR-A-2 536 301 (N.V. BEKAERT) <br> * Claims 1,3,8 * <br> --- | 6,13,14 | |
| Y | US-A-3 738 350 (A.B. STILES) <br> * Claim 1 * <br> --- | 8,9 | |
| X | FR-A-2 395 067 (KIRBY) <br> * Claims; example 1 * | 1 | |
| Y | | 6,8 | |
| | --- | | |
| Y | US-A-4 062 872 (J.Y. LEW) <br> * Claim 1 * <br> --- | 15 | TECHNICAL FIELDS SEARCHED (Int. Cl.5) |
| A | FR-A-2 250 070 (IMPERIAL CHEMICAL INDUSTRIES) <br> ----- | | B 01 J |

The present search report has been drawn up for all claims

| Place of search | Date of completion of the search | Examiner |
|---|---|---|
| THE HAGUE | 29-12-1989 | THION M.A. |